# EUROPEAN PATENT SPECIFICATION

(11) **EP 1 781 292 B1**
(45) Date of publication and mention of the grant of the patent: **22.09.2010**
(21) Application number: 05761109.7
(22) Date of filing: 13.07.2005
(51) Int. Cl.: A61K 31/495, A61K 9/20, A61P 25/00

(54) **EXTENDED RELEASE FORMULATION OF 3-AMINO-8-(1-PIPERAZINYL)-2H-1BENZOPYRAN-2-ONE**
FORMULIERUNG VON 3-AMINO-8-(1-PIPERAZINYL)-2H-1BENZOPYRAN-2-ON MIT VERZÖGERTER FREISETZUNG
PREPARATION A LIBERATION PROLONGEE DE 3-AMINO-8-(1-PIPERAZINYL)-2H-1BENZOPYRAN-2-ONE

(30) Priority: 15.07.2004 EP 04103378; 15.07.2004 US 588036 P
(43) Date of publication of application: 09.05.2007
(73) Proprietor: Abbott Healthcare Products B.V., 1381 CP Weesp (NL)
(72) Inventor: BRUINS, Nico Solvay Pharmaceuticals B.V., NL-1381 CP WEESP (NL); FRIJLINK, Henderik W. Solvay Pharmaceuticals B.V., NL-1381 CP Weesp (NL); DE VRIES, Michiel H. Solvay Pharmaceuticals B.V., NL-1381 CP Weesp (NL)
(74) Representative: Verhage, Marinus
(86) International application number: PCT/EP2005/053341
(87) International publication number: WO 2006/005760

(56) References cited:
- EP-A- 0 650 964
- WO-A-98/42344
- US-A- 5 478 572
- US-A- 6 010 717
- US-B1- 6 312 717

## Description

The present invention relates to extended release formulations of the compound 3-amino-8-(1-piperazinyl)-2H-1-benzopyran-2-one or pharmaceutically acceptable forms thereof. The invention further relates to the preparation of these extended release formulations and their use in the prevention or treatment of disorders, such as CNS disorders and pain.

3-amino-8-(1-piperazinyl)-2H-1-benzopyran-2-one is disclosed in EP 0650964 (Example 8) and is a selective 5HT_{1A} agonist and 5HT_{1D} antagonist that may be used in the treatment of CNS disorders such as anxiety and depression. The compound has the following structure:

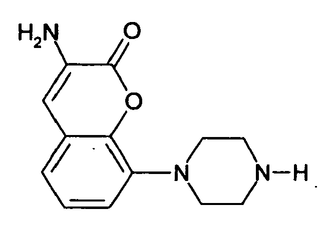

During further development of said compound in the mentioned indications, it appeared that the compound when administered as an immediate release formulation has the serious drawback of high incidence of serious nausea and vomiting at the intended clinical dose range.
It is the object of the present invention to provide a solution for this problem, as the incidence found for nausea and vomiting was prohibitive for the further development of an oral formulation of the compound.

This object can be achieved, according to the present invention by an extended release formulation of 3-amino-8-(1-piperazinyl)-2H-1-benzopyran-2-one or a pharmaceutically acceptable salt thereof.

In the framework of the present invention and in line with the European Pharmacopoeia an extended release formulation (alternatively named as a prolonged release formulation) is defined as a modified release dosage form of - which the release of the active substance and its subsequent absorption are prolonged in comparison with a conventional non modified (immediate-release) dosage form administered by the same route. A modified release formulation is defined as a formulation wherein the rate and/or place of release of the active substance is different from that of an immediate release formulation administered by the same route. In an immediate release formulation more than 75% of the active substance is released within 45 minutes. In an oral extended release formulation according to the present invention at most 40 % of the active substance is released within 45 minutes.

Several 5HT_{1A} agonists, i.e. buspirone and gepirone, have low clinical effectiveness and side-effects relating to rapid metabolism, leading to the formation of 1-2(2-pyrimidinyl)piperazine (1-PP). This problem can be solved according to WO 98/42344 by the use of a fast-dispersing dosage form that gives pre-gastric absorption. Other solutions are the concurrent administration of a 5HT_{1A} autosomal receptor antagonist such as pindolol (US 6,312,717) or the use of an extended release formulation (US 5,478,572).

Due to fact that 3-amino-8-(1-piperazinyl)-2H-1-benzopyran-2-one originates from a totally different structure class, side effects of this compound are not related to a rapid metabolization and/or the formation of a large amount of one principal metabolite.

WO 96/09815 describes the use of a special starch excipient generally indicated as "long chain amylose" for the preparation of controlled release formulations of drugs and indicates that controlled release formulations can be used to reduce side effects and to realize a low dosage of drugs in general. There is no indication, however, that a controlled release formulation can be used for the reduction of nausea and vomiting and to increase the dosage.

3-amino-8-(1-piperazinyl)-2H-1-benzopyran-2-one is known to be useful in treating and/or preventing anxiety, depression, pain and movement disorders and can be administered in different salt forms. It is effective over a wide dosage range. The term "therapeutically effective amount" in the framework of the present invention refers to an amount capable of diminishing the adverse symptoms of a particular disease. Preferred dosage is in the range of from about 0.01 to about 20 mg per day. In the treatment of adult humans a range of about 0.1 to about 2.5 mg in single or divided doses is preferred. The particular dose of compound administered according to the present invention will, however, be determined by the particular circumstances surrounding the case, including the weight, the age, the severity of the symptoms and the individual response of the patient and will also depend on the route of administration. Therefore the above mentioned dosage range are not intended to limit the scope of the present invention in any way.

3-amino-8-(1-piperazinyl)-2H-1-benzopyran-2-one may be administered in different ways. The present invention is related to an oral extended release formulation. As illustrated in Example 8 the use of an oral extended release formulation reduces the incidence of nausea and vomiting in comparison with an immediate release formulation. Oral extended release formulations may be prepared according to any method known in the art for the manufacture of extended pharmaceutical compositions.

In one embodiment the formulations may be tablets which may contain the active ingredient in admixture with pharmaceutically acceptable excipients, such as gelling agents as described below, binding agents (e.g. starch, acacia gum, gelatin), lubricating agents (e.g. stearic acid, magnesium stearate, sodium stearyl fumarate (Pruv®), glycerol behenate (Compritol®), talc), glidants (e.g. colloidal silicon dioxide (Aerosil®)) and inert diluents (e.g. calcium phosphate, sodium phosphate, calcium carbonate, sodium carbonate, mannitol, lactose and ethylcellulose), colorants, anti-oxidants, flavoring agents, sweeting agents and preservatives. In further embodiments extended release formulations may also be soft gelatin or hard gelatin capsules wherein the active ingredient is mixed with auxiliary materials causing an extended release profile or sprayed upon a core followed by coating with a material causing extended release. Further auxiliary materials may be an oily medium such as liquid paraffin, peanut oil, or olive oil, or solid fats or waxes (e.g. hydrogenated castor oil or triglycerides) an inert solid diluent such as kaolin, calcium carbonate or calcium phosphate.

Cellulose ethers are well known in the art and are available as hydrophilic and hydrophobic polymers in pharmaceutical grades and with different average molecular weights leading to different viscosities of a solution of these cellulose ethers. For the purpose of this patent application, hydrophilic polymers may be characterized by their viscosities in a 2% w/w aqueous solution as low viscosity ( less than about 1000 mPas), medium viscosity (about 1000 mPas to about 10,000 mPas) and high viscosity (greater than about 10,000 mPas).

Hydrophilic hydroxypropyl methylcellulose polymers (HPMC's) which may be used in the present invention are available in different viscosity grades from Dow Chemical Co. under the brand name Methocel® and from Shin Etsu under the brand name Metolose®.
Examples of low viscosity polymers are Methocel E5®, Methocel E-15LV®, Methocel E50LV®, Methocel K100LV® and Methocel F50LV®, whose 2% aqueous solutions at 25°C have viscosities of 5 mPas, 15 mPas, 50 mPas, 100 mPas and 50 mPas, respectively.
Examples of medium viscosity HPMC's are Methocel E4M® and Methocel K4M, whose 2% aqueous solutions at 25°C have viscosities of 4000 mPas.
Examples of high viscosity HPMC's are Methocel K15M® and Methocel K100M® whose 2% aqueous solutions at 25 C have viscosities of 15,000 mPas and 100,000 mPas.

In a preferred embodiment of the formulation according to the present invention a combination of a low viscosity HPMC and a medium viscosity HPMC is used to prepare extended release tablets. Most preferred is the combination of a HPMC with a viscosity of 5 mPas and a HPMC with a viscosity of 4000 mPas is used.

Next to the HPMC, filler binder materials may be added to the tablet formulation in order to improve tablet properties such as strength and friability. Preferably filler binder materials are chosen that have no or only limited effect on the drug release. Examples of said materials are ethylcellulose (EC) or calcium phosphate.

In an even more preferred first embodiment of the present invention a combination of low viscosity HPMC and medium viscosity HPMC is used. De ratio between medium viscosity HPMC and low viscosity HPMC is between 100 : 1 and 10:1, preferably between 20 : 1 and 15 : 1 and most preferred around 17 : 1.

In a second preferred embodiment of the present invention a special starch excipient generally indicated as "long chain amylose" is used. This type of material is described in patent WO 96/09815 and is characterized by a content of long-chain amylose (with a degree of polymerization (DP) of at least 100) between 20 and 80% by weight based on dry substance, a cold water-solubility of at most 25% by weight and a specific area of at least 1 m²/g. A more preferred material has content (w/w) of long-chain amylose of between between 50 and 70% (w/w) based on dry substance The most preferred long chain amylose to be used in the present invention is characterized by having a specific surface area of at least 6 m²/g and content of long-chain amylase of at least 60% by weight based on dry substance With cold water in the framework of the present invention is meant a temperature of between approximately 15 and approximately 25 °C.

Also in the second preferred embodiment other auxiliary materials such as binding agents (e.g. starch, acacia gum, gelatin), lubricating agents (e.g. stearic acid, magnesium stearate, sodium stearyl fumarate (Pruv®), glycerol behenate (Compritol®), talc), glidants (e.g. colloidal silicon dioxide (Aerosil®)) and inert diluents (e.g. mannitol and lactose), colorants, anti-oxidants, flavoring agents, sweeting agents and preservatives.

The following examples are only intended to further illustrate the invention, in more detail, and therefore these examples are not deemed to restrict the scope of the invention in any way.

### Example 1. Synthesis of 3-amino-8-(1-piperazinyl)-2H-1-benzopyran-2-one and its monohydrochloric acid monohydrate

### Step 1: Nitration

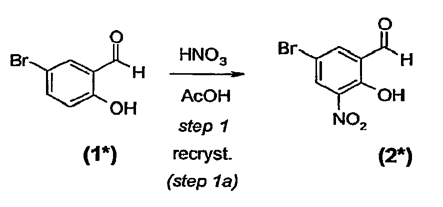

A solution of 1.0 mol of 5-bromo-2-hydroxybenzaldehyde **(1*)** in 3.75 litres acetic acid (98%) was formed on heating the mixture to about 60°C. 1.5 mol of concentrated nitric acid (137 g = 97 ml) was added slowly in approximately 1 hour. After the completion of the addition stirring was continued at 65°C for a further 10 minutes. The solution was then cooled to 45°C, and the product was precipitated by the addition of 4 litres of water. After stirring for at least 3 hours the product was collected on a filter and washed with water until the pH of the mother liquor was approximately 6. The material is dried as much as possible by centrifugation. The crude product was dissolved in 800 ml acetone under refluxing and stirring. 400 ml acetone was removed by distillation. After cooling to 20°C, the mixture was stirred for 3 hours. The precipitate was collected on a filter and washed with petroleum ether 40 -65°C. The solid was dried overnight in an air stream at 40°C. Finally, the crude **(2*)** was recrystallized from acetone to yield an end product with a purity of 98% as shown by NMR analysis.
5-bromo-2-hydroxybenzaldehyde **(1*)** was identified by its characteristic chemical shift δ 9.84 ppm; 5-bromo-2-hydroxy-3-nitrobenzaldehyde **(2*)** had a characteristic chemical shift of δ 10.4 ppm. The overall yield of this step was approximately 60% (crude on crude).

### Step 2: Erlenmeyer condensation

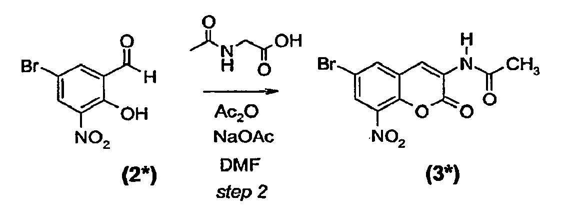

To a mixture of 1.0 mol of 5-bromo-2-hydroxy-3-nitrobenzaldehyde **(2*)**, 1.0 mol of N-acetylglycine and 1.0 mol of anhydrous sodium acetate, 800 ml of N -methyl-2-pyrrolidone are added. The mixture was stirred and heated to 50 °C. Then 2.2 mol of acetic anhydride was run into the reaction vessel in approximately 30 minutes. The reaction mixture was heated to 100°C. During heating the reacting mixture became homogeneous for a while; shortly afterwards a solid was formed, making stirring troublesome. After heating at 100°C for 4 hours, the mixture was cooled to 80°C and 1,100 ml of acetic acid (98%) was added. Thereafter stirring of the mixture was easy. Next, the mixture was cooled to room temperature, and stirred for 60 minutes. The precipitate was collected on a filter and washed twice with 625 ml acetic acid (80%), five times with 900 ml water, and once with 300 ml acetone. The product was dried in an air stream at 40°C for 24 hours, and had a purity of 98% as shown by NMR analysis.
5-bromo-2-hydroxy-3-nitrobenzaldehyde **(2*)** had a characteristic shift of δ 10.4 ppm; the characteristic chemical shift of N -(6-bromo-8-nitro-2-oxo-2H-1-benzopyran-3-yl)acetamide **(3*)** was δ 8.72 ppm. The overall yield of this step was approximately 80% (crude on crude).

### Step 3: Reduction

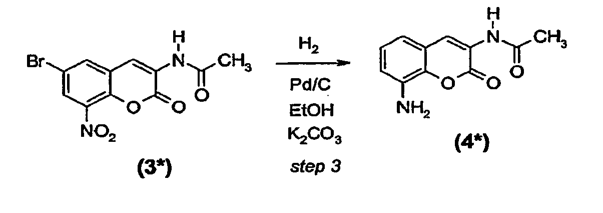

A mixture of 1.0 mol of N-(6-bromo-8-nitro-2-oxo-2H-1-benzopyran-3-yl)acetamide **(3*)**, 50 g of 10% palladium on carbon paste (containing 61% water), 1.0 mol of potassium carbonate and 15 litre of ethanol was heated to 60°C. At this temperature the starting material was reduced with hydrogen at an overpressure of 4 bar at 1400 rpm. After completion of the reaction (1 hour), the catalyst was removed by filtration using filter aid, and washed with 4.5 litre methyl ethyl ketone (MEK). The filtrate was concentrated to 2 litre, and 2.3 litre of MEK was added In order to change the solvent from ethanol to MEK, 2 litre of the solvent mixture was distilled off at normal pressure and 2 litre of MEK was added. This was repeated 4 times. Then 5 litre of MEK and 2.6 litre of water were added and the mixture was stirred. The layers were separated. The upper layer was concentrated at normal pressure to approximately 3.5 litre. The residue was cooled to 25°C. During this cooling the product crystallized. Then the mixture was cooled to -10°C and stirred for two hours. The solid was filtered and washed three times with 800 ml hexane. The product was dried (50°C, 20 cm Hg, N₂) until constant weight.

N-(6-bromo-8-nitro-2-oxo-2H-1-benzopyran-3-yl)acetamide **(3*)** had a characteristic chemical shift of δ 8.72 ppm; that of N-(8-amino-2-oxo-2H-1-benzo-pyran-3-yl)-acetamide (4) was δ 8.55 ppm. The overall yield of this step was approximately 70% (crude on crude).

### Step 4: Formation of the piperazine ring

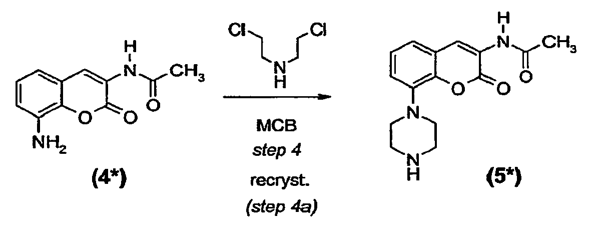

A mixture of 2.5 litre monochlorobenzene, 1.0 mol of N-(8-amino-2-oxo-2H-1-benzopyran-3-yl)-acetamide **(4*)** and 1.2 mol bischloroethylamine hydrochloride was heated to reflux under nitrogen. Part of the monochlorobenzene (0.5 litre) was distilled off. This mixture was refluxed for 10 days. The reaction was followed by HPLC. After the reaction, the mixture was cooled to 20°C and stirred overnight. The solid product was collected on a filter and washed once with 360 ml monochlorobenzene and 3 times with 360 ml ethanol. The product was dried in vacuum at 50°C.
Half of the crude product was dissolved in 3 litre water. After addition of 18 g of Celite and 50 g of charcoal, the mixture was stirred for 1 hour at room temperature. After filtration the solution was concentrated by distillation of water. In the mean time the second half of the crude product was treated as described above. When the total volume of the combined aqueous solutions was about 1.5 litre, distillation was stopped and the mixture was cooled to room temperature. Then 125 g sodium bicarbonate was added in portions. After stirring for 1.5 hours at 15°C the precipitate formed was collected on a filter. After washing with 360 ml water and 2 times with 270 ml ethanol, the product was dried in vacuum at 50 °C. N-(8-amino-2-oxo-2H-1-benzo-pyran-3-yl)-acetamide **(4*)** had a characteristic chemical shift of δ 8.55 ppm; that of N-(8-(1-piperazinyl)-2-oxo-2H-1-benzopyran-3-yl-)acetamide **(5*)** was δ 8.57 ppm. The overall yield of this step was approximately 50% (crude on crude).

### Step 5: Amide hydrolysis

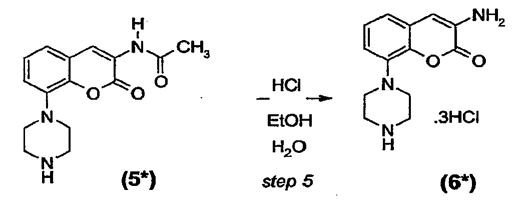

2.9 Litre of concentrated hydrochloric acid was added at room temperature to a suspension of 1.0 mol of N-(8-(1-piperazinyl)-2-oxo-2H-1-benzopyran-3-yl-) acetamide **(5*)** and 1.4 litre of absolute ethanol in about 10 minutes. During this addition the temperature rose to 40°C. After the addition the mixture was stirred at a temperature of 50°C during 1.5 hours. The mixture was cooled to 20°C and, after crystallisation had started, 1.4 litre of absolute ethanol was added. Then the mixture was stirred for 1 hour at 20°C and for 2 hours at 0°C. The crystals were isolated by filtration and washed twice with 0.6 litre of acetone. The isolated product was dried in vacuum (40°C, 200 mm Hg, N₂, 24 hours).

N-(8-(1-piperazinyl)-2-oxo-2H-1-benzopyran-3-yl-)acetamide **(5*)** had a characteristic chemical shift of δ 8.57 ppm; the trihydrochloric acid salt of 3-amino-8-(1-piperazinyl)-2H-1-benzopyran-2-one **(6*)** had a characterristic chemical shift of δ 6.77 ppm. The overall yield of this step was approximately 85% (crude on crude).

### Step 6: Partial neutralisation

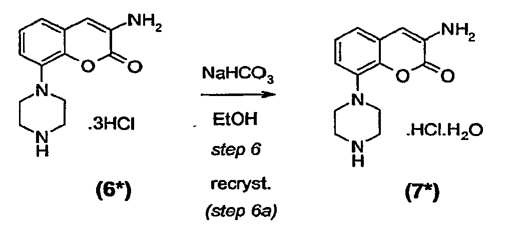

To a suspension of 1.0 mol of the trihydrochloric acid salt **(6*)** In 3.5 litre ethanol a solution of 2.2 mol sodium bicarbonate in 2.8 litre water was added in about 30 minutes. The temperature was between 20°C and 25°C. The suspension was then stirred for 3 hours. The reaction mixture was filtered and subsequently washed with 1.1 litre water, 1.1 litre ethanol and 1.1 litre hexane. The isolated crude product was dried in vacuum (40°C, 200 mm Hg, N₂, 24 hours).

The dried product (1 mol) was dissolved in 9 litre methanol by heating to reflux temperature. The solution did not become completely dear. After cooling to 20 °C the mixture was filtered. 300 ml of water and 150 ml of methanol was added to the filtrate, after which about 3 litre of the solvent mixture was distilled at normal pressure. The complete procedure was repeated with another mol of the dried product. Then the combined fractions wre concentrated to a volume of about 12 litre by distillation. After addition of 6 litre ethanol, 6 litre of the solvent mixture was removed by distillation at normal pressure. The mixture was then cooled to 0°C and stirred for 2 hours. The precipitate was collected on a filter and washed twice with 750 ml acetone. The product was dried under vacuum (40°C, 200 mm Hg, N₂, 24 hours), and thereafter homogenized by milling and, when necessary, by micronizing.

The trihydrochloric acid salt of 3-amino-8-(1-piperazinyl)-2H-1-benzopyran-2-one **(6*)** had a characteristic chemical shift of δ 6.77 ppm; that of the end product, 3-amino-8-(1-piperazinyl)-2H-1-benzopyran-2-one monohydrochloric acid monohydrate, was δ 6.7 ppm. The overall yield of this step was approximately 85% (crude on crude). 3-amino-8-(l-piperazinyl)-2H-1-benzopyran-2-one monohydrochloric acid monohydrate, had a molecular formula C₁₃H₁₈ClN₃O₃ and a molecular mass of 299.5. The pure product (99.8%, NMR) was a white to yellowish powder. Its chloride content was 11.7% (mass to mass), as determined by titrimetry. Its water content, determined by Karl Fisher water assay titration, was 6.5% (mass to mass).

### Example 2. Preparation of an extended release formulation of 3-amino-8-(1-piperazinyi)-2H-1-benzopyran 2-one (HPMC based).

Tablets with strengths of 0.50 and 0.75 mg were prepared according to the following procedures (required quantities are given in Table 1).
Hydroxypropylmethylcellulose (I) was sieved and, if necessary, moistened with water and granulated. This granular material was dried and broken. The required quantities of 3-amino-8-(1-piperazinyl)-2H-1-benzopyran-2-one (as monohydrate of the hydrochloride salt), hydroxypropylmethylcellulose (II) and ethylcellulose were sieved, if necessary, and mixed with the screened granules. The colloidal anhydrous silica and glyceryl behenate were sieved, if necessary, and mixed with the granular mixture. This final granular material was compressed into tablets of about 120 mg. The products, having the composition indicated in Table 2 were packed and tested. In order to obtain the material for the double blind study described in Example 3, the tablets were filled into capsules: one 0.50 mg tablet in a capsule or a combination of a 0.50 mg and a 0.75 mg tablet in a capsule.

**Table 1. Required amount of active ingredient and auxiliary materials for a batch size of 100 000 extended release tablets of 0.50 mg or 0.75 mg tablets of 3-amino-8-(1-piperazinyl)-2H-1-benzopyran-2-one**

| tablet strength in mg ¹⁾ | 0.50 | 0.75 |
|---|---|---|
| Components | per batch of 100 000 tablets (in g) | |
| active ingredient as monohyd rate of HCl salt | 61.1 | 91.7 |
| hydroxypropylmethylcellulose (I) ²⁾ | 524 | 522 |
| hydroxypropylmethylcellulose (II) ³⁾ | 8867 | 8838 |
| ethylcellulose ⁴⁾ | 2339 | 2339 |
| colloidal anhydrous silica | 60 | 60 |
| glyceryl behenate | 150 | 150 |
| purified water about | 340 | 340 |

**Table 2. Theoretical formula per tablet**

| tablet strength in mg ¹⁾ | 0.50 | 0.75 |
|---|---|---|
| Components | quantities in mg | |
| active ingredient as monohydrate of HCl salt | 0.611 | 0.917 |
| hydroxypropylmethylcellulose (I)²⁾ | 5.24 | 5.22 |
| hydroxypropylmethylcellulose (II)³⁾ | 88.67 | 88.38 |
| Ethylcellulose ⁴⁾ | 23.39 | 23.39 |
| colloidal anhydrous silica | 0.6 | 0.6 |
| glyceryl behenate | 1.5 | 1.5 |
| purified water | - | - |

| | | |
|---|---|---|
| ¹⁾ as base ²⁾ labelled viscosity: 5 mPa s (2% mN solution in water) ³⁾ labelled viscosity: 4000 mPa s (2% m/V solution in water) ⁴⁾ labelled viscosity: 5.5 mPa s (5% m/V solution in toluene/ethanol) | | |

### Example 3. Preparation of an extended release formulation of 3-amino-8-(1-piperazinyl)-2H-1-benzopyran-2-one (long chain amylose based)

3-amino-8-(1-piperazinyl)-2H-1-benzopyran-2-one (as monohydrate of the hydrochloride salt) was dry mixed with a special long-chain amylose as disclosed in WO 96/09815, which material is characterized by a content of long-chain amylase of at least 60% by weight based on the dry substance, a cold water solubility of at most 10% by weight and a specific surface area of at least 6 m²/g and glyceryl tribehenate according to the composition given in table 3. Mixing was done in a turbula mixer containing all components for 30 minutes. This mixture was dry granulated and compressed on a diaf TM20 tabletting press into tablets of 120 mg, with a diameter of 6.5 mm and a crushing strength above 150 N. The tablets contained 800 microgram of 3-amino-8-(1-piperazinyl)-2H-1-benzopyran-2-one (calculated as base). In another experiment the quantitative composition of the tablets was changed according to table 3. This composition was compressed into tablets of 70 mg , with a diameter of 5.0 mm and a crushing strength above 150 N. Again the tablets contained 800 microgram of 3-amino-8-(1-piperazinyl)-2H-1-benzopyran-2-one (calculated as base).

**Table 3: Composition of slow release tablets based on long-chain amylose**

| | | |
|---|---|---|
| Tablet weight in mg | 120 | 70 |
| Tablet diameter in mm | 6.5 | 5.0 |
| Components: | Relative amount: | Relative amount |
| 3-amino-8-(1-piperazinyl)-2H-1benzopyran-2-one HCl.H₂O | 0.81 % | 1.39 % |
| Amylose (long-chain)(as described above) | 96.69 % | 96.11 % |
| Glyceryl tribehenate | 2.5 % | 2.5 % |

### Example 4. Preparation of an immediate release formulation of 3-amino-8-(1-piperazinyl)-2H-1-benzopyran-2-one.

Capsules with strengths of 0.10, 0.20 and 0.50 mg were prepared according to the following procedures (required quantities are given in Table 4).
About 95% of the mannitol was stirred in a mixer. The required quantity of 3-amino-8-(1-piperazinyl)-2H-1-benzopyran-2-one (as monohydrate of the hydrochloride salt) was mixed with the remainder of the mannitol and then mixed with the mannitol in the mixer. The glyceryl behenate and sodium starch glycollate were added to the mixture and mixed. This final powder mixture was filled into capsules, size no. 2, on an encapsulating machine. The filling weight of a capsule is about 240 mg, but may be adjusted depending on the content of 3-amino-8-(1-piperazinyl)-2H-1-benzopyran-2-one in the powder mixture. The product, having the composition indicated in Table 5, was packed and tested.

**Table 4. Required amount of active ingredient and auxiliary materials for a batch size of 4000 capsules of 0.10 mg, 0.20 or 0.50 mg immediate release capsules of 3-amino-8-(1-piperazinyl)-2H-1-benzopyran-2-one.**

| Capsule strength in mg (as base) | 0.1 | 0.2 | 0.5 |
|---|---|---|---|
| Components | Per batch of 4000 capsules (in g) | | |
| active ingredient as monohydrate of HCl salt | 0.488 | 0.976 | 2.444 |
| Sodium starch glycollate | 38.4 | 38.4 | 38.4 |
| glyceryl behenate | 19.2 | 19.2 | 19.2 |
| Mannitol | 902 | 901.6 | 900 |
| hard gelatin capsule, opaque blue, size no. 2 | 4000 | 4000 | 4000 |

**Table 5. Theoretical formula per capsule**

| Capsule strength in mg (as base) | 0.1 | 0.2 | 0.5 |
|---|---|---|---|
| Components | quantities in mg | | |
| active ingredient as monohydrate of HCl salt | 0.122 | 0.244 | 0.611 |
| Sodium starch glycollate | 9.6 | 9.6 | 9.6 |
| glyceryl behenate | 4.8 | 4.8 | 4.8 |
| Mannitol | 225.5 | 225.4 | 225.0 |

### Example 5. In vitro dissolution of extended release tablets of 3-amino-8-(1-piperazinyl)-2H-1-benzopyran-2-one as prepared in Example 2.

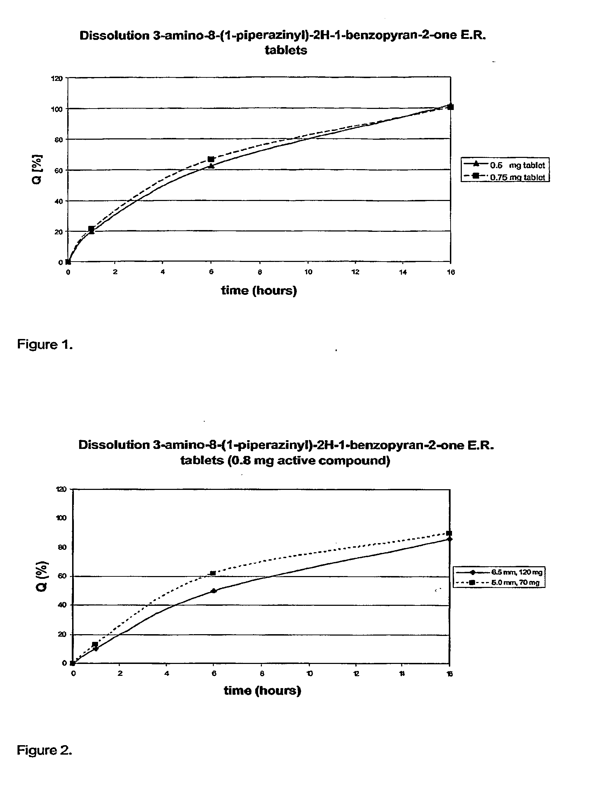

In-vitro dissolution study of 6 tablets was performed with the USP apparatus II, using an aqueous buffer solution of pH 4.7 as release medium and a rotation speed of the paddle of 75 rpm. Table 6 gives the results of this test (also depicted in Figure 1).

**Table 6. Results of invitro dissolution of extended release tablets prepared according to Example 2**

| tablet strength in mg ¹⁾ | 0.50 | 0.75 |
|---|---|---|
| Time in hours | Release of active substance in % of total | |
| 0 | 0 | 0 |
| 1 | 20 | 22 |
| 6 | 63 | 67 |
| 16 | 102 | 101 |

| | | |
|---|---|---|
| ¹⁾ as base | | |

### Example 6. In vitro dissolution of extended release tablets of 3-amino-8-(1-piperazinyl)-2H-1-benzopyran 2-one as prepared in Example 3.

In-vitro dissolution study of 6 tablets was performed with the USP apparatus II, using an aqueous buffer solution of pH 4.7 as release medium and a rotation speed of the paddle of 75 rpm. Table 7 gives the results of this test (also depicted in Figure 2):

**Table 7. Results of in vitro dissolution of extended release tablets prepared according to Example 3**

| | | |
|---|---|---|
| tablet strength in mg ¹⁾ | 0.8 | 0.8 |
| tablet weight in mg | 120 | 70 |
| tablet diameter in mm | 6.5 | 5.0 |

| Time in hours | Release of active substance in % of total | |
|---|---|---|
| 0 | 0 | 0 |
| 1 | 10 | 13 |
| 6 | 50 | 62 |
| 16 | 86 | 90 |

| | | |
|---|---|---|
| ¹⁾ as base | | |

### Example 7. In vitro dissolution of immediate release tablets of 3-amino-8-(1piperazinyl)-2H-1-benzopyran-2-one as prepared in Example 4.

In-vitro dissolution study of 6 tablets was performed with the USP apparatus II, using an aqueous buffer solution of pH 4.7 as release medium and a rotation speed of the paddle of 75 rpm Table 8 gives th e results of this test.

**Table 8. Results of in vitro dissolution of immediate release capsules prepared according to Example 4**

| Capsule strength in mg¹ | 0.1 | 0.2 | 0.5 |
|---|---|---|---|
| Time in minutes | Release of active substance in % of total | | |
| 15 | 99 | 96 | 97 |
| 30 | 100 | 97 | 97 |
| 45 | 100 | 97 | 98 |

| | | | |
|---|---|---|---|
| ¹⁾ as base | | | |

### Example 8. Incidence of nausea and vomiting after application of an extended release formulation in comparison with application of an immediate release formulation.

3-amino-8-(1-piperazinyl)-2H-1-benzopyran-2-one was given as immediate release (IR) formulations prepared according to Example 4 or as extended release (ER) tablets prepared according to Example 2 in single and multiple dose studies to healthy male volunteers in the age range of 18 to 43 years. From a total of 156 subjects 104 received one or more single doses and 52 subjects received multiple doses of 3-amino-8-(1-piperazinyl)-2H-1-benzopyran-2-one. In the multiple dose studies 3-amino-8-(1-piperazinyl)-2H-1-benzopyran-2-one was administered for a period of 7 days. Adverse events information was assessed at regular intervals after single and multiple doses using non-leading questions.

The occurrence of nausea after single doses, which was one of the most frequently reported adverse events with an immediate release formulation, was reported from single doses of 0.4 mg of 3-amino-8-(1-piperazinyl)-2H-1-benzopyran-2-one onwards. Vomiting occurred in about half of the subjects who reported nausea.
The expected clinical dose range is between 0.5 and 2.5 mg daily. As a consequence, tolerance problems might prohibit that patients can be dosed high enough with an immediate release formulation to realize any efficacy.

Table 9 and 10 clearly show that the use of the extended release formulation leads to much fewer reports of nausea and vomiting. Also, higher dose levels can be given before these symptoms do occur.
The percentage of subjects reporting nausea after single doses of 0.4, 0.6 and 0.8 mg 3-amino-8-(1-piperazinyl)-2H-1-benzopyran-2-one given as IR formulation was 8 %, 16 % and 50 % respectively, while for vomiting these figures were 6 %, 4 % and 17 % respectively. However, after single doses of 0.5 and 1.25 mg as ER formulation these figures were 0 % and 11 % respectively for nausea and 0 % for vomiting after both dose levels.
A similar picture is seen after multiple doses. The percentage of subjects reporting nausea after multiple doses of 0.4, 0.8 and 1.2 mg 3-amino-8-(1-piperazinyl)-2H-1-benzopyran-2-one once daily as IR formulation was 17 %, 75 % and 50 % respectively, while no nausea was reported after dosing with 1.25 mg ER formulation and 33% after 2.5 mg ER tablets. No vomiting was seen at these dose regimens except at the highest dose level given (1.25 mg ER).

**Table 9. Number of subjects (% between brackets) reporting nausea and vomiting after different single dose levels of 3-amino-8-(1-piperazinyl)-2H-1-benzopyran-2-one given as immediate (IR) and as extended (ER) release formulations.**

| **Dose (mg)** | **Pla** | **0.4 IR** | **0.6 IR** | **0.8 IR** | **0.5 ER** | **1.25 ER** |
|---|---|---|---|---|---|---|
| Number of subjects | 61 | 36 | 67 | 12 | 4 | 18 |
| Nausea | 3 | 3 | 11 | 6 | 0 | 2 |
| | (5%) | (8%) | (16%) | (50%) | (0%) | (11%) |
| Vomiting | 0 | 2 | 3 | 2 | 0 | 0 |
| | (0%) | (6%) | (4%) | (17%) | (0%) | (0%) |

**Table 10. Number of subjects (% between brackets) reporting nausea and vomiting after different multiple dose levels of 3-amino-8-(1-piperazinyl)-2H-1-benzopyran-2-one given once daily as immediate (IR) and as extended (ER) release formulations.**

| **Dose (mg)** | **Placebo** | **0.4 IR** | **0.8 IR** | **1.2 IR** | **1.25 ER** | **2.5 ER** |
|---|---|---|---|---|---|---|
| Number of subjects | 11 | 6 | 4 | 4 | 8 | 10 |
| Nausea | 0 | 1 | 3 | 2 | 0 | 3 |
| | (0%)- | (17%) | (75%) | (50%) | (0%) | (33%) |
| Vomiting | 0 | 0 | 0 | 0 | 0 | 1 |
| | (0%) | (0%) | (0%) | (0%) | (0%) | (10%) |

In conclusion it can be stated that the extended release formulation of 3-amino-8-(1-piperazinyl)-2H-1-benzopyran-2-one is associated with much less nausea and vomiting as the immediate release formulation. As a consequence higher dose levels of 3-amino-8-(1-piperazinyl)-2H-1-benzopyran-2-one can be given with the extended release formulation.

## Claims

1. An extended release formulation comprising:
a) an amount in the range of about 0.1 to maximally about 2.5 mg of 3-amino-8-(1-piperazinyl)-2H-1-benzopyran-2-one, or a pharmaceutically acceptable salt thereof, or a hydrate of said pharmaceutically acceptable salt thereof,
b¹) a hydroxypropylmethyl cellulose, or,
b²) a mixture of two or more hydroxypropylmethyl celluloses, or,
b³) a starch excipient comprising between 20 and 80%—by weight based on dry substance-long-chain amylose, having a degree of polymerization of at least 100, a cold water-solubility of at most 25% by weight, and a specific area of at least 1 m²/g,
said extended release formulation releasing at most 40% of the active substance within 45 minutes.

2. An extended release formulation according to claim 1, wherein said mixture of two or more hydroxypropylmethyl celluloses comprises a low viscosity hydroxypropylmethyl cellulose, and a medium viscosity hydroxypropylmethyl cellulose.

3. An extended release formulation according to claim 1, wherein said starch excipient comprises at least 60%—by weight based on dry substance—long-chain amylose, having a specific area of at least 6 m²/g.

4. An extended release formulation according to any one of the claims 1-3, wherein said pharmaceutically acceptable salt is a mono hydrochloride salt.

5. An extended release formulation according to claim 4, wherein said hydrate is a monohydrate.

6. Use of the formulation according to any one of the claims 1-5 for the preparation of a medicament for the treatment of CNS disorders or pain.

7. Use according to claim 6, wherein the CNS disorder is chosen from depression, anxiety and movement disorders.

## Patentansprüche

1. Formulierung mit verlängerter Freisetzung, welche umfasst:
a) eine Menge im Bereich von etwa 0,1 bis maximal etwa 2,5 mg von 3-Amino-8-(1-piperazinyl)-2H-1-benzopyran-2-on, oder ein pharmazeutisch annehmbares Salz davon, oder ein Hydrat von besagtem pharmazeutisch annehmbaren Salz davon,
b¹) eine Hydroxypropylmethylcellulose oder
b²) ein Gemisch aus zwei oder mehr Hydroxypropylmethylcellulosen oder
b³) einen Stärke-Arzneimittelträger, welcher zwischen 20 und 80 Gew.-%, basierend auf Trockensubstanz, langkettige Amylose mit einem Polymerisationsgrad von mindestens 100, einer Löslichkeit in kaltem Wasser von höchstens 25 Gew.-% und einer spezifischen Oberfläche von mindestens 1 m²/g umfasst,
wobei besagte Formulierung mit verlängerter Freisetzung höchstens 40% des Wirkstoffs innerhalb von 45 Minuten freisetzt.

2. Formulierung mit verlängerter Freisetzung gemäß Anspruch 1, worin besagtes Gemisch aus zwei oder mehr Hydroxypropylmethylcellulosen eine Hydroxypropylmethylcellulose von niedriger Viskosität und eine Hydroxypropylmethylcellulose von mittlerer Viskosität umfasst.

3. Formulierung mit verlängerter Freisetzung gemäß Anspruch 1, worin besagter Stärke-Arzneimittelträger mindestens 60 Gew.-%, basierend auf Trockensubstanz, langkettige Amylose mit einer spezifischen Oberfläche von mindestens 6 m²/g umfasst.

4. Formulierung mit verlängerter Freisetzung gemäß einem der Ansprüche 1-3, worin besagtes pharmazeutisch annehmbares Salz ein Monohydrochloridsalz ist.

5. Formulierung mit verlängerter Freisetzung gemäß Anspruch 4, worin besagtes Hydrat ein Monohydrat ist.

6. Verwendung der Formulierung gemäß einem der Ansprüche 1-5 für die Herstellung eines Medikaments zur Behandlung von ZNS-Störungen oder Schmerz.

7. Verwendung gemäß Anspruch 6, wobei die ZNS-Störung ausgewählt ist aus Depression, Angstzuständen und Bewegungsstörungen.

## Revendications

1. Formulation à libération prolongée comprenant :
a) une quantité comprise dans la fourchette d'environ 0,1 à environ 2,5 mg maximum de 3-amino-8-1-(1-pipérazinyl)-2H-1-benzopyran-2-one, ou un sel pharmaceutiquement acceptable de celle-ci ou un hydrate dudit sel pharmaceutiquement acceptable,
b¹) une hydroxypropylméthylcellulose, ou
b²) un mélange de deux hydroxypropylméthylcelluloses ou plus, ou
b³) un excipient de type amidon comprenant entre 20 et 80 %, en poids par rapport au poids de la substance sèche, d'amylose à chaîne longue, ayant un degré de polymérisation d'au moins 100, une solubilité dans l'eau froide d'au plus 25 % en poids et une surface spécifique d'au moins 1 m²/g,
ladite formulation à libération prolongée libérant au maximum 40 % de la substance active en l'espace de 45 min.

2. Formulation à libération prolongée selon la revendication 1, où ledit mélange de deux hydroxypropylméthylcelluloses ou plus comprend une hydroxypropylméthylcellulose à faible viscosité et une hydroxypropylméthylcellulose à viscosité moyenne.

3. Formulation à libération prolongée selon la revendication 1, où ledit excipient de type amidon comprend au moins 60 % en poids par rapport à la substance sèche d'amylose à chaîne longue ayant une surface spécifique d'au moins 6 m²/g.

4. Formulation à libération prolongée selon l'une quelconque des revendications 1 à 3, où ledit sel pharmaceutiquement acceptable est un sel de type monochlorhydrate.

5. Formulation à libération prolongée selon la revendication 4, où ledit hydrate est un monohydrate.

6. Utilisation de la formulation selon l'une quelconque des revendications 1 à 5 pour la préparation d'un médicament destiné au traitement des troubles du SNC et des douleurs.

7. Utilisation selon la revendication 6 où le trouble du SNC est choisi parmi la dépression, l'anxiété et les troubles des mouvements.
